(19) Europäisches Patentamt
European Patent Office
Office européen des brevets

(11) **EP 4 685 454 A1**

(12) **EUROPEAN PATENT APPLICATION**

(43) Date of publication:
**28.01.2026 Bulletin 2026/05**

(21) Application number: **25190357.1**

(22) Date of filing: **18.07.2025**

(51) International Patent Classification (IPC):
**G01N 1/30** (2006.01)     **G01N 15/10** (2024.01)
**G01N 15/14** (2024.01)

(52) Cooperative Patent Classification (CPC):
**G01N 15/1484; G01N 1/30; G01N 15/1459;**
G01N 2015/1006; G01N 2015/1486

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **23.07.2024 JP 2024117954**

(71) Applicant: FUJIFILM Corporation
**Tokyo 106-8620 (JP)**

(72) Inventors:
• **KASAI, Yusuke**
**Ashigarakami-gun, Kanagawa, 258-8577 (JP)**
• **MORIMOTO, Takashi**
**Ashigarakami-gun, Kanagawa, 258-8577 (JP)**
• **YUKAWA, Hiroyuki**
**Ashigarakami-gun, Kanagawa, 258-8577 (JP)**
• **WAKABAYASHI, Akira**
**Ashigarakami-gun, Kanagawa, 258-8577 (JP)**
• **OBA, Takahiro**
**Ashigarakami-gun, Kanagawa, 258-8577 (JP)**

(74) Representative: **Hoffmann Eitle**
**Patent- und Rechtsanwälte PartmbB**
**Arabellastraße 30**
**81925 München (DE)**

(54) **PARTICLE DETECTION DEVICE AND PARTICLE DETECTION METHOD**

(57)     Provided are a particle detection device and a particle detection method that have high particle identification properties, can ensure sterility, and have automation suitability.

A particle detection device includes a holding part that holds a suspension containing particles, a first flow channel that is connected to the holding part, an observation window that is connected to the first flow channel, a liquid feeding part that transfers the suspension held in the holding part to the observation window, and a fluorescence detection part that detects fluorescence emitted from the particles contained in the suspension through the observation window.

FIG. 1

EP 4 685 454 A1

## Description

## BACKGROUND OF THE INVENTION

1. Field of the Invention

The disclosed technology relates to a particle detection device and a particle detection method.

2. Description of the Related Art

Regarding a technology of detecting particles such as cells, the following technology is known. For example, JP1995-082008B (JP-H07-082008B) discloses a diagnostic apparatus including image enlargement means for enlarging an image of a cell, live cell image recognition means for recognizing a living cell from the image obtained by the image enlargement means, and dead cell image recognition means for recognizing a dead cell. The dead cell image recognition means comprises staining means for staining a cell in a low activity state.

WO2016/013395A discloses a cell counter including a flow channel through which a cell suspension flows, liquid drive means for feeding the cell suspension in the flow channel, and measurement means for measuring the number of cells contained in the cell suspension flowing in the flow channel over time while mixing the cell suspension using the liquid drive means.

JP2005-241435A discloses a method of counting live cells, such as microorganisms or cell tissues, in a sample by labeling the live cells with a fluorescent reagent to measure the number of the live cells. This method involves exciting live cells labeled with a fluorescent reagent with light of a specific short wavelength, measuring the amount of fluorescence emitted from the live cells at a wavelength relatively longer than the short wavelength, and calculating the number of the live cells based on correlation between the number of the live cells per unit area and a measured fluorescence amount value, which is obtained in advance.

## SUMMARY OF THE INVENTION

With the recent development of regenerative medicine, automation of cell processing and culture processing has progressed. Basically, cell culture is carried out so that the number of cells reaches a target number, regardless of whether the purpose is for research or for production. Therefore, it is considered that counting the number of cells is essential in the automation of the cell processing and the culture processing. In general, the counting of the number of cells includes a step of manually sampling a small amount of cell suspension from the parent suspension. Since this step is performed in an open system, issues arise with respect to sterility and automation suitability. In addition, the counting of the number of cells is often performed by bright-field microscopic observation using white light. However, in a case in which the cell suspension contains particles other than cells, it is difficult to distinguish the cells from the other particles in the bright-field microscopic observation, and it is difficult to accurately count the number of cells.

The disclosed technology has been made in view of the above points, and an object of the disclosed technology is to provide a particle detection device and a particle detection method that have high particle identification capability, can ensure sterility, and have automation suitability.

A particle detection device according to the disclosed technology comprises: a holding part that holds a suspension containing particles; a first flow channel that is connected to the holding part; an observation window that is connected to the first flow channel; a liquid feeding part that transfers the suspension held in the holding part to the observation window; and a fluorescence detection part that detects fluorescence emitted from the particles contained in the suspension through the observation window.

The particle detection device may further comprise: a mixing part in which the suspension flowing through the first flow channel and a fluorescent reagent are mixed. The mixing part may have a second flow channel provided between the first flow channel and the observation window. A solid-phase fluorescent reagent may be attached to an inner wall of the second flow channel. The mixing part may have a form of a cartridge including the observation window and the second flow channel.

The particle detection device may further comprise: a connector that has a first port to which the first flow channel is connected and a second port to which the second flow channel is connected, and to which the cartridge of the mixing part is attachably and detachably attached. The particle detection device may further comprise: a valve that opens and closes the first flow channel.

The liquid feeding part may include a pump connected to the first flow channel on an upstream side of the mixing part. The particle detection device may further comprise a first valve that opens and closes the first flow channel on an upstream side with respect to a connection point between the first flow channel and the pump, and a second valve that opens and closes the first flow channel on a downstream side with respect to the connection point and on an upstream side with respect to the mixing part.

The particle detection device may further comprise: a counting part that counts the number of particles detected by the fluorescence detection part. The particles to be detected may be cells.

A particle detection method according to the disclosed technology comprises: transferring a suspension containing particles held in a holding part to an observation window via a first flow channel using a liquid feeding part; and detecting fluorescence emitted from the particles contained in the suspension through the observation window.

The suspension and a fluorescent reagent may be mixed in a mixing part connected to the first flow channel. The liquid feeding part may include a pump connected to the first flow channel on an upstream side of the mixing part. In this case, the suspension may be transferred to the observation window while at least one of a first valve that opens and closes the first flow channel on an upstream side with respect to a connection point between the first flow channel and the pump or a second valve that opens and closes the first flow channel on a downstream side with respect to the connection point and on an upstream side with respect to the mixing part is maintained in a closed state. The particles to be detected may be cells.

According to the disclosed technology, a particle detection device and a particle detection method that can ensure sterility and have automation suitability are provided.

**BRIEF DESCRIPTION OF THE DRAWINGS**

Fig. 1 is a diagram schematically showing an example of a configuration of a particle detection device according to an embodiment of the disclosed technology.

Fig. 2 is a cross-sectional view schematically showing an example of a configuration of a mixing part according to the embodiment of the disclosed technology.

Fig. 3 is a diagram schematically showing another example of the configuration of the particle detection device according to the embodiment of the disclosed technology.

Fig. 4A is a perspective view showing an example of a configuration of a connector and a cartridge according to the embodiment of the disclosed technology.

Fig. 4B is a perspective view showing a state where a flow channel and the cartridge are connected to the connector according to the embodiment of the disclosed technology.

Fig. 4C is a cross-sectional view of the connector in a state where the flow channel and the cartridge are connected.

Fig. 5A is a perspective view showing another example of the configuration of the connector according to the embodiment of the disclosed technology.

Fig. 5B is a front view of the connector as viewed in a direction indicated by an arrow in Fig. 5A.

Fig. 5C is a perspective view showing another example of the configuration of the connector according to the embodiment of the disclosed technology.

Fig. 5D is a side view of the connector according to the embodiment of the disclosed technology.

Fig. 5E is a front view of the connector according to the embodiment of the disclosed technology.

Fig. 6 is a diagram showing an example of a specific flow channel configuration of the particle detection device according to the embodiment of the disclosed technology.

Fig. 7 is a diagram showing an example of a configuration of a tube assembly according to the embodiment of the disclosed technology.

Fig. 8A is a diagram showing an example of a procedure for transferring a suspension according to the embodiment of the disclosed technology.

Fig. 8B is a diagram showing an example of a procedure for transferring the suspension according to the embodiment of the disclosed technology.

Fig. 8C is a diagram showing an example of a procedure for transferring the suspension according to the embodiment of the disclosed technology.

Fig. 8D is a diagram showing an example of a procedure for transferring the suspension according to the embodiment of the disclosed technology.

Fig. 8E is a diagram showing an example of a procedure for transferring the suspension according to the embodiment of the disclosed technology.

Fig. 9 is a diagram showing another example of the specific flow channel configuration of the particle detection device according to the embodiment of the disclosed technology.

Fig. 10 is a diagram showing an example of a configuration of a tube assembly according to the embodiment of the disclosed technology.

Fig. 11A is a diagram showing an example of a procedure for transferring the suspension according to the embodiment of the disclosed technology.

Fig. 11B is a diagram showing an example of a procedure for transferring the suspension according to the embodiment of the disclosed technology.

Fig. 11C is a diagram showing an example of a procedure for transferring the suspension according to the embodiment of the disclosed technology.

Fig. 11D is a diagram showing an example of a procedure for transferring the suspension according to the embodiment of the disclosed technology.

**DESCRIPTION OF THE PREFERRED EMBODIMENTS**

Hereinafter, an example of an embodiment of the disclosed technology will be described with reference to the drawings. The same or equivalent components and parts in the drawings will be denoted by the same reference numerals, and repeated description thereof will be omitted.

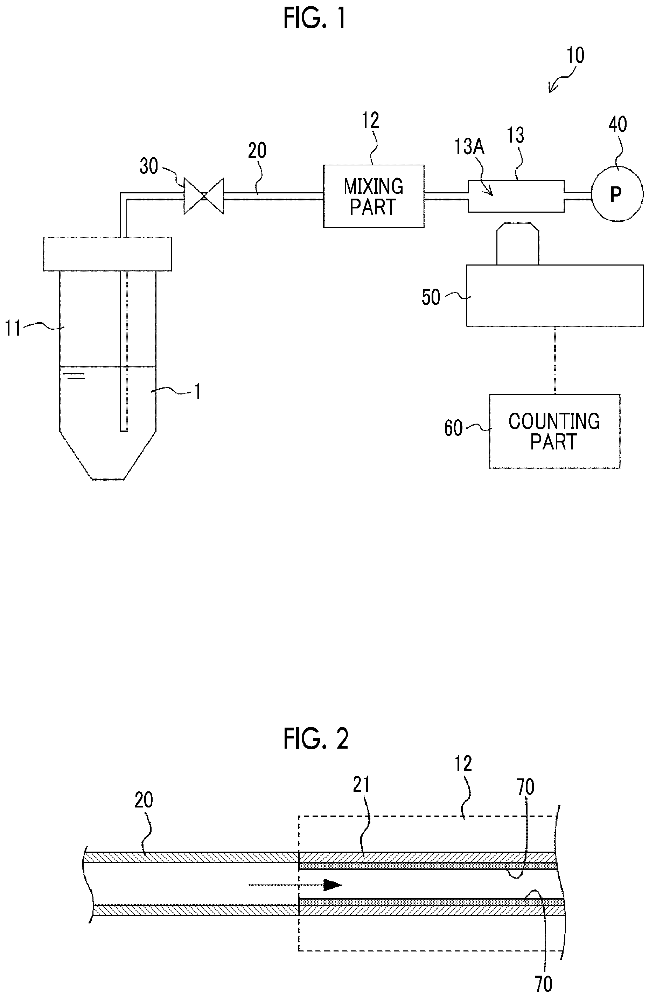

Fig. 1 is a diagram schematically showing an example of a configuration of a particle detection device 10 according to an embodiment of the disclosed technology. The particle detection device 10 includes a holding part 11, a flow channel 20, a valve 30, a mixing part 12, an observation window 13, a liquid feeding part 40, a fluorescence detection part 50, and a counting part 60. The particle detection device 10 has a function of detecting particles contained in a suspension 1 held in the holding part 11. The particles to be detected are, for example, cells. The cell may be a single cell or may have a morphology of a colony or spheroid formed by aggregation of a plurality of cells.

The holding part 11 is a member for holding the suspension 1 containing the particles to be detected by the particle detection device 10. The holding part 11 may have, for example, a form of a centrifuge tube or a cell culture vessel. The flow channel 20 is connected to the holding part 11, and the suspension 1 held in the holding part 11 flows through the flow channel 20. The flow channel 20 may be formed of, for example, a tube. The flow channel 20 is an example of a "first flow channel" in the disclosed technology.

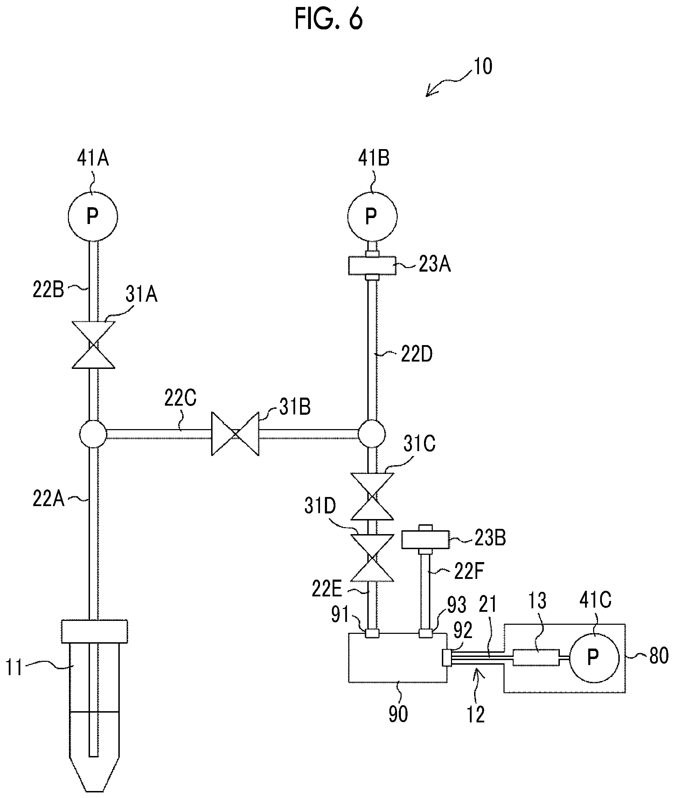

The flow channel 20 is provided with the valve 30. The valve 30 opens and closes the flow channel 20. By providing the valve 30, the upstream side and the downstream side of the valve 30 can be separated. As a result, for example, even in a case in which a fluorescent reagent included in the mixing part 12 has toxicity, it is possible to prevent a toxic component from flowing to the upstream side of the valve 30. In addition, by closing the valve 30, it is possible to perform liquid feeding on the downstream side of the valve 30 without affecting the upstream side of the valve 30. As a result, it is possible to increase the degree of freedom in design of the downstream side of the valve 30. In addition, by adopting a two-stage configuration of the valve as shown in Fig. 6, it is possible to maintain a sterile state in a section on the upstream side even in a case in which a section on the most downstream side is in a non-sterile state.

The mixing part 12 is connected to the flow channel 20. The suspension 1 flowing through the flow channel 20 passes through the mixing part 12, whereby the suspension 1 is mixed with the fluorescent reagent. The cells, which are particles contained in the suspension 1, are stained with the fluorescent reagent.

Fig. 2 is a cross-sectional view schematically showing an example of a configuration of the mixing part 12. The mixing part 12 has a flow channel 21 connected to the flow channel 20. The flow channel 20 and the flow channel 21 may be connected via a connector (not shown). A solid-phase fluorescent reagent 70 is attached to an inner wall of the flow channel 21 of the mixing part 12. The cell suspension passes through the flow channel 21, whereby the solid-phase fluorescent reagent 70 is dissolved and mixed with the cell suspension. The fluorescent reagent 70 has a function of selectively fluorescently staining cells. The fluorescent reagent 70 may include, for example, acridine orange (AO) and 4',6-diamidino-2-phenylindole (DAPI). AO is used for staining all cells, and DAPI is used for staining dead cells. Particles (for example, oil droplets derived from a biological tissue) other than the cells contained in the cell suspension are not stained with the fluorescent reagent 70. The flow channel 21 is an example of a "second flow channel" in the disclosed technology. The fluorescent reagent may be a liquid, and the mixing part 12 may be configured to mix the liquid fluorescent reagent with a suspension.

The observation window 13 has a pair of parallel flat plates having light transmittance. A chamber 13A formed between the pair of parallel flat plates communicates with the flow channel 21 of the mixing part 12. The suspension mixed with the fluorescent reagent in the mixing part 12 is accommodated in the chamber 13A. The particles contained in the suspension accommodated in the chamber 13A can be observed from the outside.

The liquid feeding part 40 is liquid feeding means for transferring the suspension 1 held in the holding part 11 to the observation window 13. The liquid feeding part 40 may include liquid feeding means such as a tube pump and a syringe pump. The number and the installation position of the liquid feeding parts 40 are not particularly limited. One or two or more liquid feeding parts 40 are installed to transfer the suspension 1 held in the holding part 11 to the observation window 13.

The fluorescence detection part 50 detects fluorescence emitted from the particles contained in the suspension 1 through the observation window 13. The fluorescence detection part 50 may have, for example, a form of a fluorescence microscope. That is, the fluorescence detection part 50 may include an excitation light source for exciting a phosphor introduced into a particle to be detected, an objective lens that magnifies an image of the particle to be detected, an imaging unit that captures the image of the particle magnified by the objective lens, and an imaging lens that forms the image of the particle magnified by the objective lens on an imaging surface of the imaging unit. The objective lens is focused on the inside of the observation window 13. The imaging unit outputs an image of the particles that emit fluorescence. The fluorescence detection part 50 may be of either an inverted type or an upright type.

The counting part 60 counts the number of particles detected by the fluorescence detection part 50. Specifically, the counting part 60 acquires the image output from the imaging unit of the fluorescence detection part 50, extracts granular objects from the acquired image, and outputs a count value of the number of the extracted objects. The counting part 60 may output a density D (the number per unit volume) of the particles by performing calculation represented by Equation (1). In Equation (1), S is a size of an image including particles to be detected, T is a thickness of the chamber 13A of the observation window 13, and C is a count value of the number of particles. In addition, the counting part 60 may output the total number A of particles by performing calculation represented by Equation (2). In Equation (2), D is the density calculated based on Equation (1), and V is a volume of the suspension 1 held in the holding part 11. The counting part 60 includes a computer that executes the above-described series of processes.

$$D = C/(S \times T) \ldots (1)$$

$$A = D \times V \ldots (2)$$

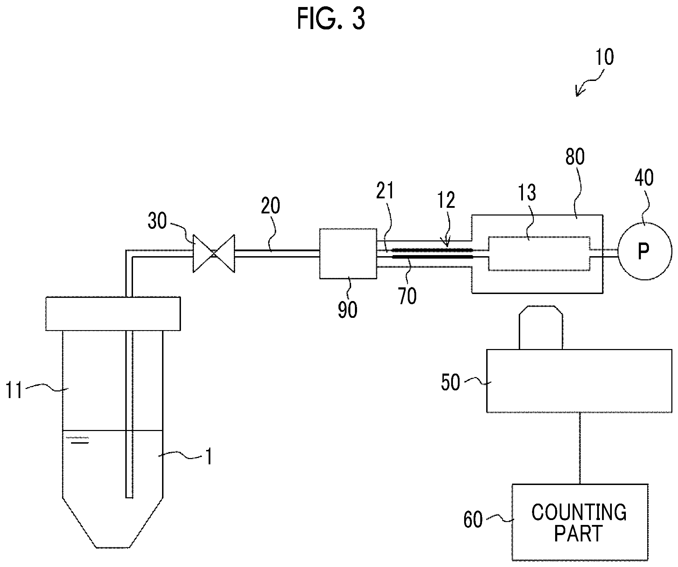

Fig. 3 is a diagram schematically showing another example of the configuration of the particle detection device 10. As shown in Fig. 3, the mixing part 12 may have a form of a cartridge 80 that integrally includes the flow channel 21 having the solid-phase fluorescent reagent 70 attached to its inner wall, and the observation window 13. The cartridge 80 is attachable to and detachable from the flow channel 20. Since the mixing part 12 has the form of the cartridge 80 that is attachable to and detachable from the flow channel 20, the mixing part 12 can be handled as a single-use member. That is, a new fluorescent reagent can be introduced into the device only by replacing the cartridge 80. As a result, it is not necessary to manage the storage of the fluorescent reagent. As shown in Fig. 3, the cartridge 80 may be attached to the device by using the connector 90. The flow channel 20 connected to the holding part 11 and the flow channel 21 in the cartridge 80 are connected via the connector 90.

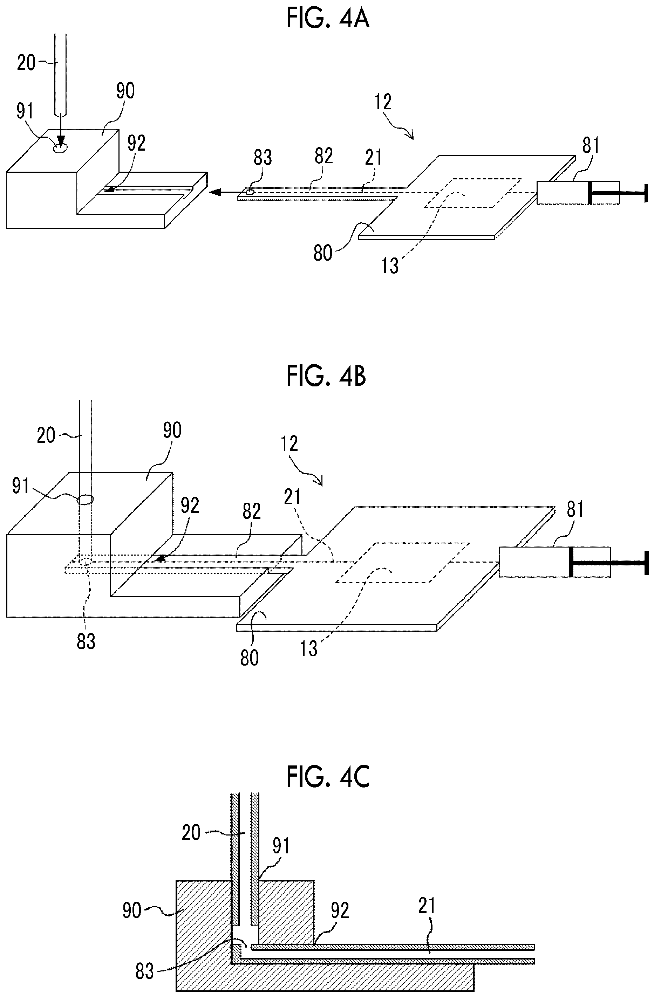

Fig. 4A is a perspective view showing an example of a configuration of the connector 90 and the cartridge 80. The cartridge 80 is a thin cassette that integrally includes the flow channel 21 having the solid-phase fluorescent reagent attached to its inner wall and the observation window 13. The flow channel 21 having the solid-phase fluorescent reagent attached to its inner wall may be meandered in order to ensure a length of the flow channel. The chamber of the observation window 13 communicates with the flow channel 21. A terminal end of the flow channel 21 extending to the downstream side of the observation window 13 is connected to a syringe pump 81. The cartridge 80 has a protrusion 82 and is connected to the connector 90 at the protrusion 82. The flow channel 21 extends to a tip of the protrusion 82, and a hole portion 83 communicating with the flow channel 21 is provided at the tip of the protrusion 82.

The connector 90 has a first port 91 to which the flow channel 20 connected to the holding part 11 is connected and a second port 92 to which the flow channel 21 in the cartridge 80 is connected. The first port 91 communicates with the second port 92. As shown in Figs. 4B and 4C, the flow channel 20 is connected to the first port 91, and the protrusion 82 of the cartridge 80 is inserted into the second port 92, thereby connecting the flow channel 20 and the flow channel 21. In a state where the flow channel 20 and the flow channel 21 are connected, the suspension in the flow channel 20 is sucked into the flow channel 21 in the cartridge 80 by pulling back a piston of the syringe pump 81. As the suspension passes through the flow channel 21, the solid-phase fluorescent reagent is dissolved, and the fluorescent reagent and the suspension are mixed. The suspension mixed with the fluorescent reagent is accommodated in the chamber of the observation window 13.

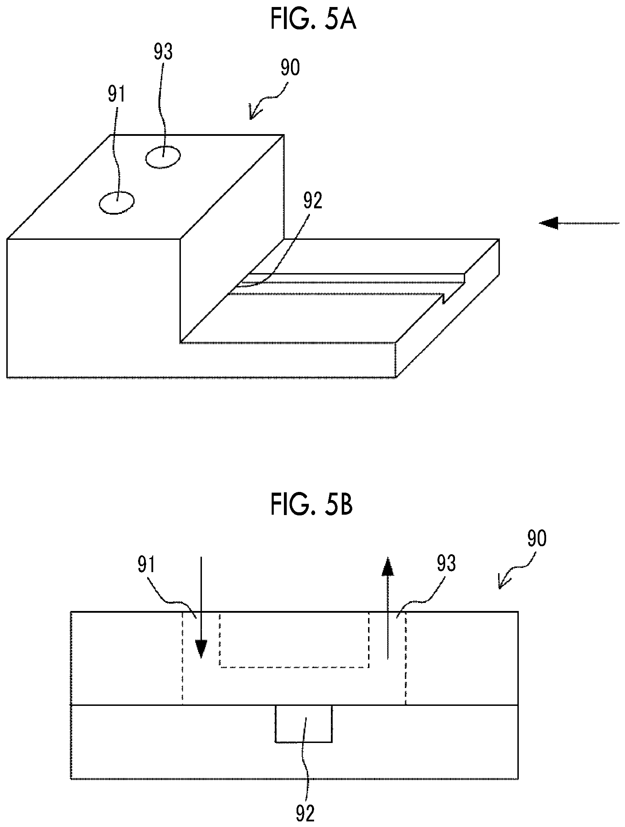

Fig. 5A is a perspective view showing another example of the configuration of the connector 90, and Fig. 5B is a front view of the connector 90 as viewed in an arrow direction in Fig. 5A. The connector 90 may have a third port 93 for venting in addition to the first port 91 and the second port 92. The first port 91 communicates with the second port 92 and the third port 93.

With the connector 90 (see Fig. 4A) that does not have the third port for venting, an internal pressure of the connector 90 increases due to air compression during liquid feeding from the flow channel 20, and leakage may occur. In order to deal with this issue, it is considered to increase the volume of an air chamber inside the connector 90, but, in this case, it is difficult to accurately add the suspension dropwise into the hole portion 83 of the cartridge 80. Since the connector 90 includes the third port 93 for venting, air flowing into the inside of the connector 90 during liquid feeding from the flow channel 20 is discharged from the third port 93, so that it is possible to suppress an increase in the internal pressure of the connector 90 during liquid feeding. It is preferable that a sterile filter is attached to a tip of an exhaust tube (not shown) connected to the third port 93.

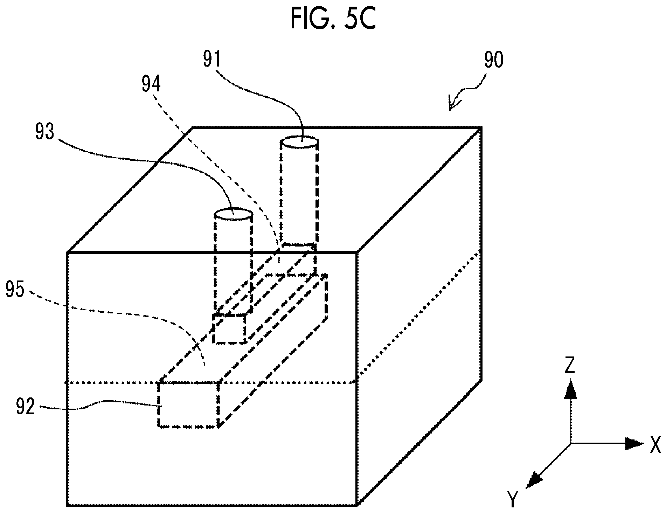

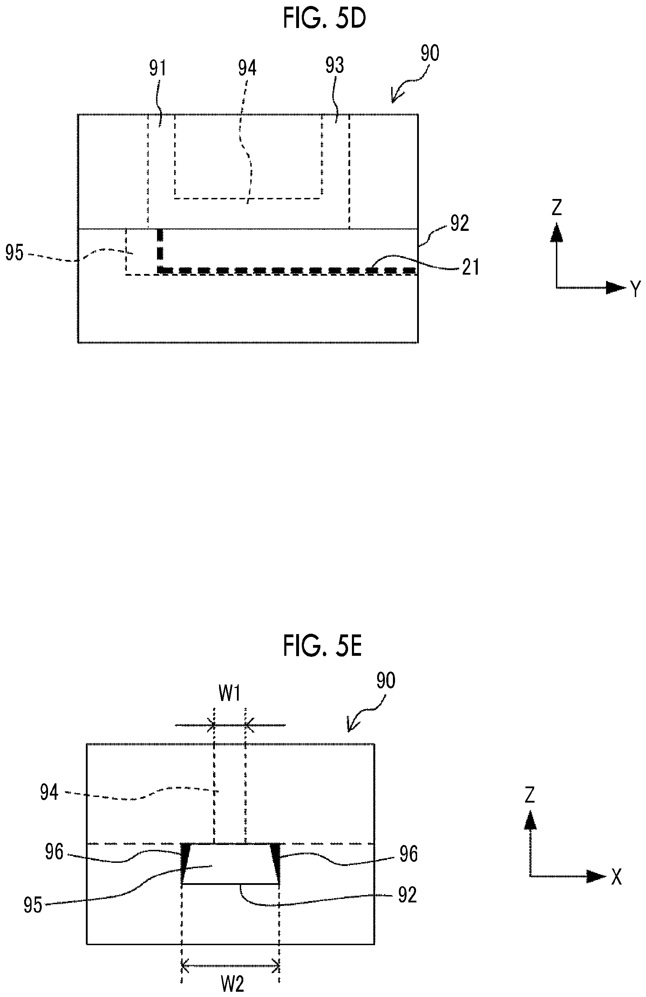

Fig. 5C is a perspective view showing another example of the configuration of the connector 90, Fig. 5D is a side view (Y-Z plane) of the connector 90 shown in Fig. 5C, and Fig. 5E is a front view (X-Z plane) of the connector 90 shown in Fig. 5C. The connector 90 has an upper cavity 94 for allowing the first port 91 and the third port 93 for venting to communicate with each other, and a lower cavity 95 into which the protrusion 82 of the cartridge 80 is inserted. An end part of the lower cavity 95 is the second port 92. A length W1 of the upper cavity 94 in an X direction is shorter than a length W2 of the lower cavity 95 in the X direction (W1 < W2), and the upper cavity 94 is disposed inside the lower cavity 95. In a case in which a clearance 96 is formed between the lower cavity 95 and the protrusion 82 of the cartridge 80 inserted into the lower cavity 95, assuming that W1 > W2, liquid may enter the clearance portion during liquid feeding, causing liquid leakage. By setting W1 < W2, it is possible to prevent liquid from entering the clearance portion, so that it is possible to prevent the occurrence of liquid leakage.

It is preferable that a material of the connector 90 is an elastic member, such as rubber, in order to ensure sealability. For example, polydimethylsiloxane (PDMS), natural rubber (NR), styrene butadiene rubber (SBR), chloroprene rubber (CR), acrylonitrile rubber (NBR), butyl rubber (IIR), ethylene propylene rubber (EPDM), urethane rubber (U), silicone rubber (Si), fluorine rubber (FKM), and chlorosulfonated polyethylene rubber (CSM) can be used as the material of the connector 90.

In addition, the material of the connector 90 may be a plastic material in order to reduce molding costs. For example, polycarbonate (PC), acrylonitrile butadiene styrene (ABS), polyoxymethylene (POM), polyphthalamide (PPA), polypropylene (PP), polystyrene (PS), polyethylene (PE), polybutylene terephthalate (PBT), and polyethylene terephthalate (PET) can be used as the material of the connector 90.

Fig. 6 is a diagram showing an example of a specific flow channel configuration of the particle detection device 10. Fig. 6 illustrates a configuration including tubes 22A, 22B, 22C, 22D, 22E, and 22F that form a flow channel for the suspension. One end of the tube 22A is connected to the holding part 11, and the other end thereof is connected to one end of each of the tubes 22B and 22C. The other end of the tube 22B is connected to a pump 41A. The pump 41A is, for example, a syringe pump. A valve 31A is provided in the middle of the tube 22B. The other end of the tube 22C is connected to one end of each of the tubes 22D and 22E. A valve 31B is provided in the middle of the tube 22C. A pump 41B is connected to the other end of the tube 22D via a sterile filter 23A. The pump 41B is, for example, a syringe pump. The other end of the tube 22E is connected to the first port 91 of the connector 90. Valves 31C and 31D are provided in the middle of the tube 22E. One end of the tube 22F is connected to the third port 93 for venting of the connector 90, and the other end thereof is connected to a sterile filter 23B. The cartridge 80 having the mixing part 12 and the observation window 13 is connected to the second port 92 of the connector 90. A pump 41C is provided on the downstream side of the observation window 13. The pump 41C is a syringe pump incorporated in the cartridge 80.

The valves 31A, 31B, and 31C are electromagnetic pinch valves that are opened and closed in response to a control signal. The valve 31D is a manual pinch valve that is opened and closed by a manual operation. The tubes can be connected using, for example, a T-shaped tube connector. The flow channel formed by the tubes 22A, 22C, and 22E is an example of a "first flow channel" in the disclosed technology. The valve 31B is an example of a "first valve" in the disclosed technology. The valve 31C is an example of a "second valve" in the disclosed technology. The pumps 41A, 41B, and 41C are examples of a "liquid feeding part" in the disclosed technology.

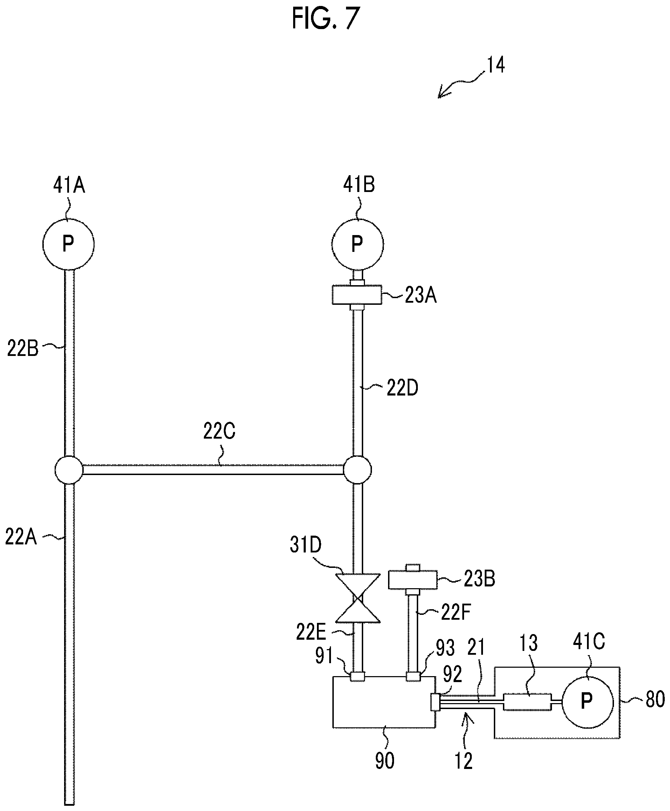

The holding part 11 and the electromagnetic valves 31A, 31B, and 31C are attached to a device housing. A tube assembly 14 (see Fig. 7) in which the tubes 22A to 22F, the pumps 41A and 41B, the sterile filters 23A and 23B, the manual valve 31D, the connector 90, and the cartridge 80 are combined is attached to the device housing, whereby the particle detection device 10 shown in Fig. 6 is configured. In a case in which the tube assembly 14 is attached to the device housing, the manual valve 31D is brought into a closed state. In the particle detection device 10 having the configuration shown in Fig. 6, the suspension is transferred by the following procedure.

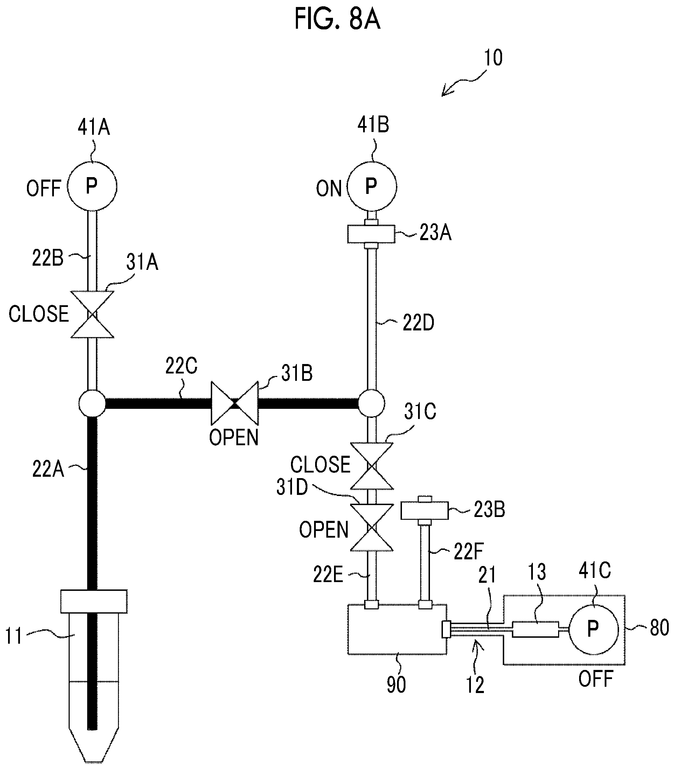

After the tube assembly 14 is attached to the device housing, the valve 31A is brought into a closed state, the valve 31B is brought into an open state, and the valve 31C is brought into a closed state. After that, the manual valve 31D is brought into an open state. Next, the pump 41B is operated to transfer the suspension from the holding part 11 to the tube 22A and the tube 22C (Fig. 8A).

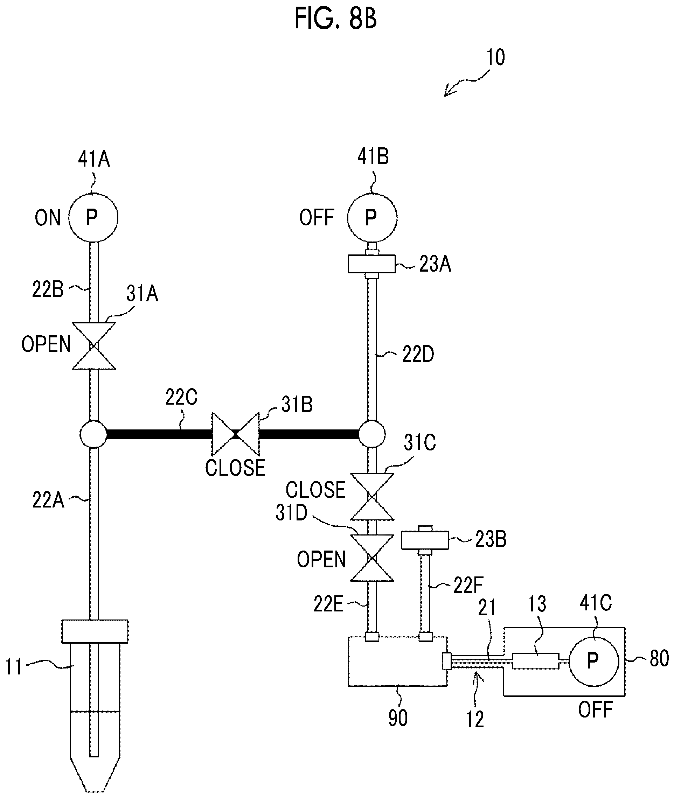

Next, the valve 31B is brought into a closed state, the valve 31A is brought into an open state, and the pump 41A is operated to restore the excess suspension to the holding part 11. As a result, the suspension remains only in the tube 22C (Fig. 8B).

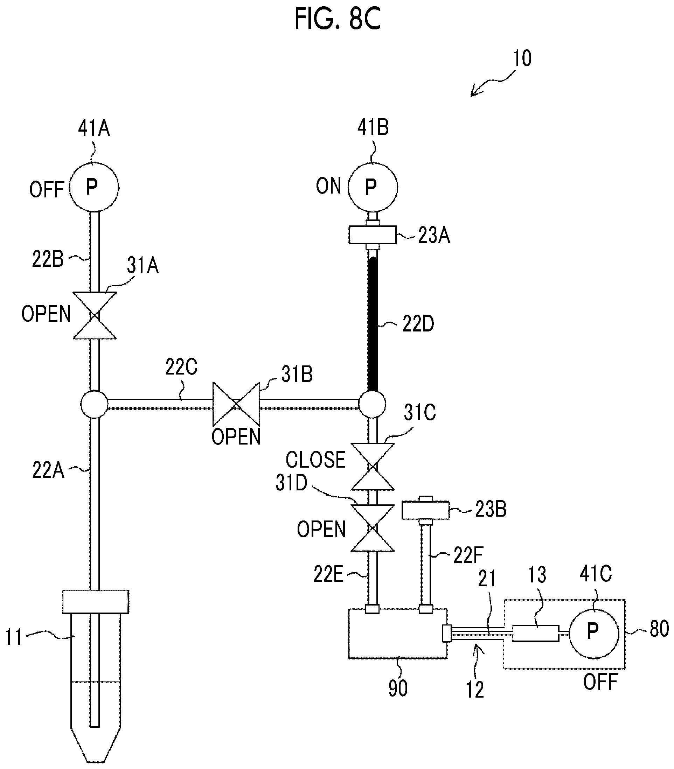

Next, the valve 31B is brought into an open state, and the pump 41B is operated to transfer the suspension from the tube 22C to the tube 22D (Fig. 8C).

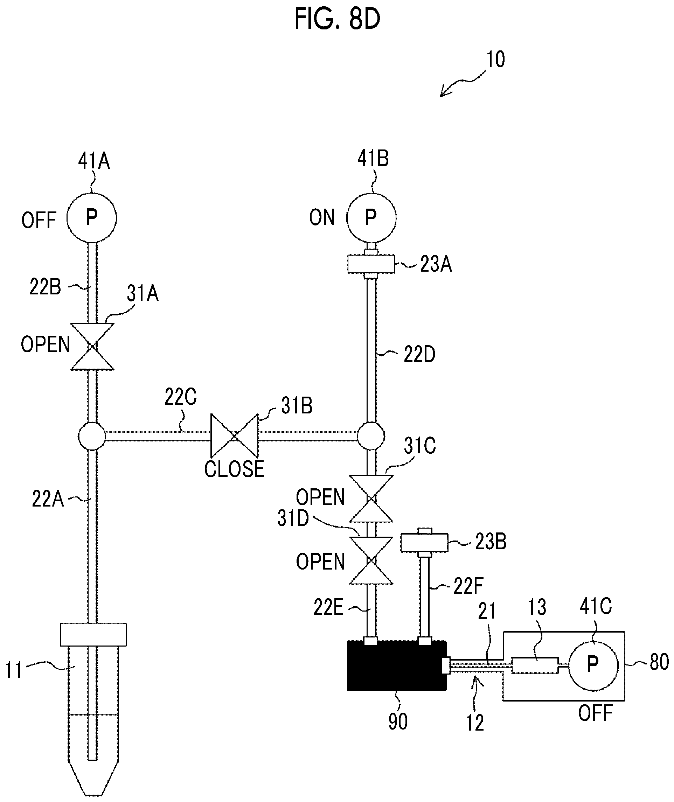

Next, the valve 31B is brought into a closed state, the valve 31C is brought into an open state, and the pump 41B is operated to transfer the suspension from the tube 22D to the inside of the connector 90 (Fig. 8D).

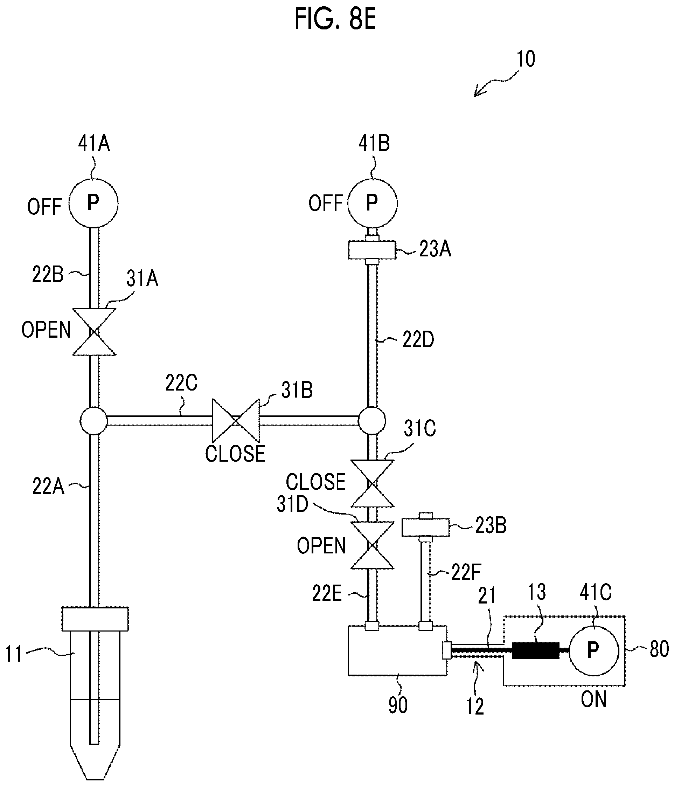

Next, the valve 31C is brought into a closed state, and the pump 41C is operated to allow the suspension to flow into the cartridge 80. As the suspension passes through the flow channel 21 in the cartridge 80, the suspension is mixed with the fluorescent reagent and is accommodated in the chamber of the observation window 13 (Fig. 8E).

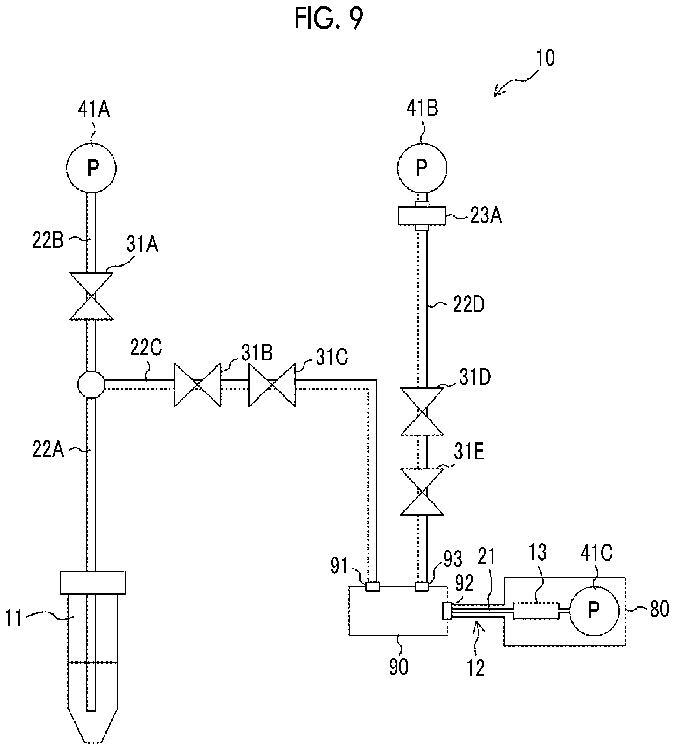

Fig. 9 is a diagram showing another example of the specific flow channel configuration of the particle detection device 10. Fig. 9 illustrates a configuration including tubes 22A, 22B, 22C, and 22D that form a flow channel for the suspension. One end of the tube 22A is connected to the holding part 11, and the other end thereof is connected to one end of each of the tubes 22B and 22C. The other end of the tube 22B is connected to a pump 41A. The pump 41A is, for example, a syringe pump. A valve 31A is provided in the middle of the tube 22B. The other end of the tube 22C is connected to the first port 91 of the connector 90. Valves 31B and 31C are provided in the middle of the tube 22C. One end of the tube 22D is connected to the third port 93 for venting of the connector 90, and the other end thereof is connected to a pump 41B via a sterile filter 23A. The pump 41B is, for example, a syringe pump. Valves 31D and 31E are provided in the middle of the tube 22D. The cartridge 80 having the mixing part 12 and the observation window 13 is connected to the second port 92 of the connector 90. A pump 41C is provided on the downstream side of the observation window 13. The pump 41C is a syringe pump incorporated in the cartridge 80.

The valves 31A, 31B, and 31D are electromagnetic pinch valves that are opened and closed in response to a control signal. The valves 31C and 31E are manual pinch valves that are opened and closed by a manual operation. The tubes can be connected using, for example, a T-shaped tube connector. The flow channel formed by the tubes 22A and 22C is an example of a "first flow channel" in the disclosed technology. The pumps 41A, 41B, and 41C are examples of a "liquid feeding part" in the disclosed technology.

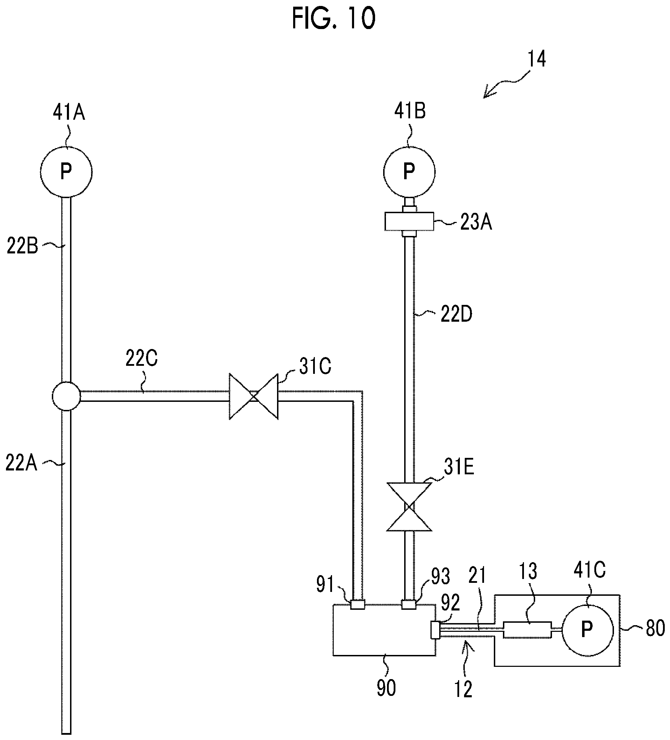

The holding part 11 and the electromagnetic valves 31A, 31B, and 31D are attached to a device housing. A tube assembly 14 (see Fig. 10) in which the tubes 22A to 22D, the pumps 41A and 41B, the sterile filter 23A, the manual valves 31C and 31E, the connector 90, and the cartridge 80 are combined is attached to the device housing, whereby the particle detection device 10 shown in Fig. 9 is configured. In a case in which the tube assembly 14 is attached to the device housing, the manual valves 31C and 31E are brought into a closed state. In the particle detection device 10 having the configuration shown in Fig. 9, the suspension is transferred by the following procedure.

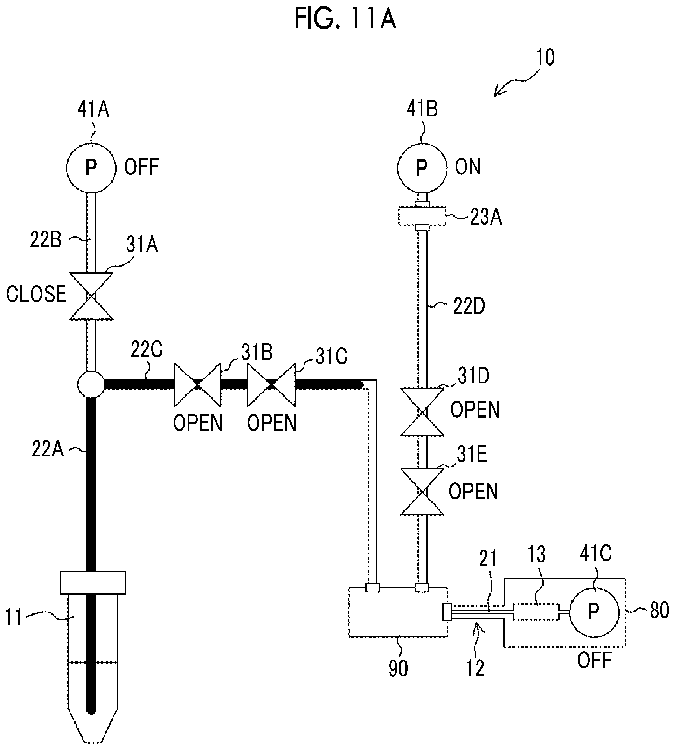

After the tube assembly 14 is attached to the device housing, the valve 31A is brought into a closed state, and the valves 31B and 31D are brought into an open state. After that, the manual valves 31C and 31E are brought into an open state. Next, the pump 41B is operated to transfer the suspension from the holding part 11 to the tube 22A and the tube 22C (Fig. 11A).

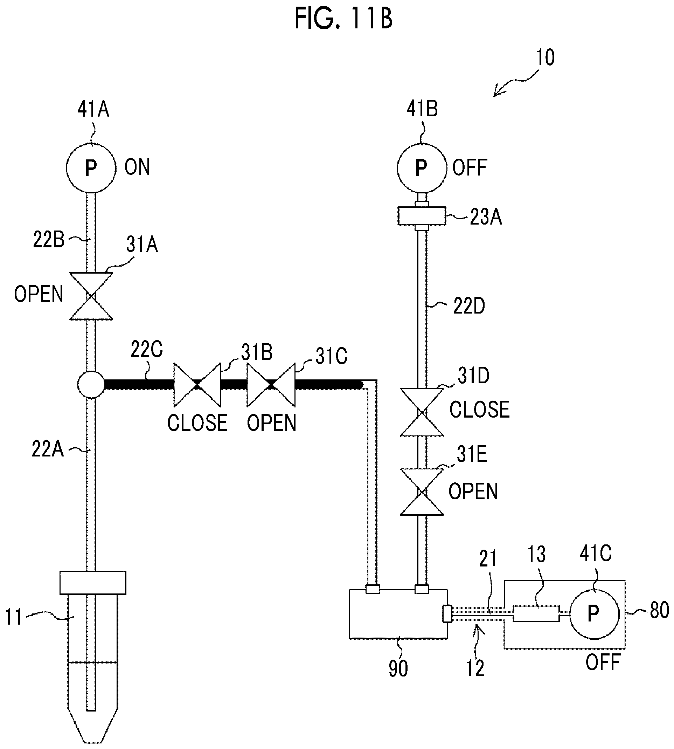

Next, the valves 31B and 31D are brought into a closed state, the valve 31A is brought into an open state, and the pump 41A is operated to restore the excess suspension to the holding part 11. As a result, the suspension remains only in the tube 22C (Fig. 11B).

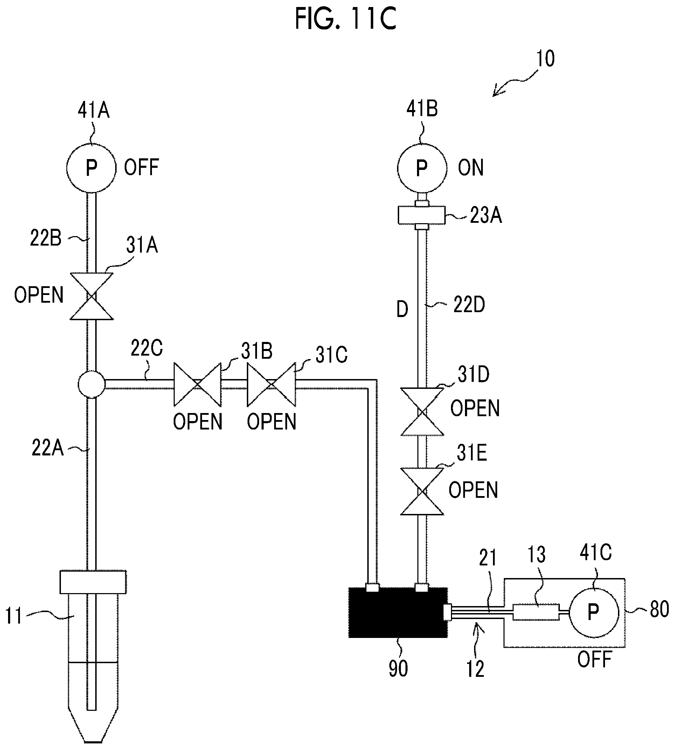

Next, the valves 31B and 31D are brought into an open state, and the pump 41B is operated to transfer the suspension from the tube 22C to the inside of the connector 90 (Fig. 11C).

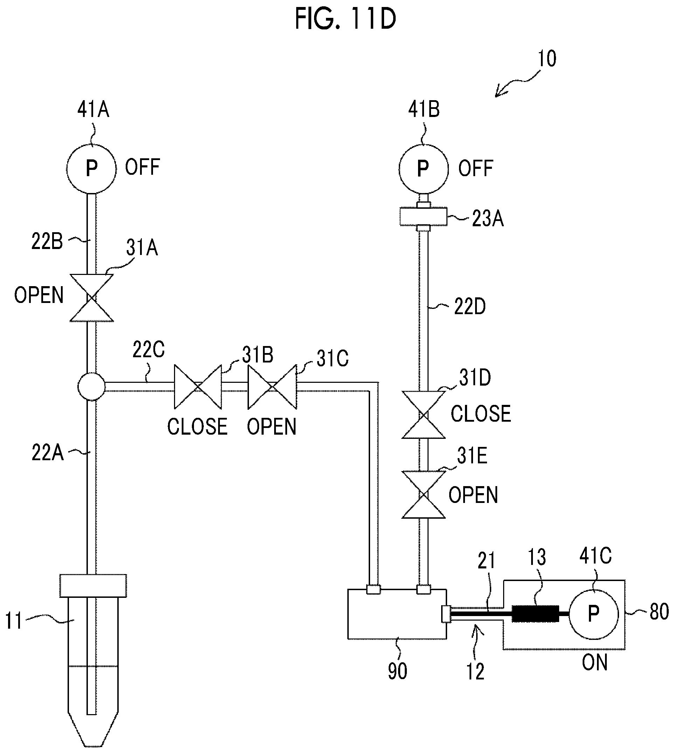

Next, the valves 31B and 31D are brought into a closed state, and the pump 41C is operated to allow the suspension to flow into the cartridge 80. As the suspension passes through the flow channel 21 in the cartridge 80, the suspension is mixed with the fluorescent reagent and is accommodated in the chamber of the observation window 13 (Fig. 11D).

According to the liquid feeding procedure in the particle detection device 10 having the flow channel configuration shown in Fig. 6, after the tube assembly 14 is attached, at least one of the valve 31B or the valve 31D provided in the middle of the flow channel from the holding part 11 to the connector 90 is maintained in a closed state until the transfer of the suspension is completed, so that the holding part 11 does not communicate with the connector 90 and the cartridge 80. Therefore, even in a case in which the connector 90 and the cartridge 80 in a non-sterilized state are used, the suspension held in the holding part 11 is maintained in a sterile state.

Incidentally, in a case in which the device is stopped in an emergency, it may be necessary to perform work of temporarily detaching the cartridge 80 from the device and then reattaching the cartridge 80 to the device. In this case, a sterile state cannot be maintained in the flow channel. With the flow channel configuration shown in Fig. 6, it is possible to transfer the suspension on the downstream side with respect to the valve 31B in a state where the valve 31B is closed. That is, it is possible to perform the liquid feeding in the event of an emergency stop without contaminating the suspension held in the holding part 11. On the other hand, the flow channel configuration shown in Fig. 6 requires a larger number of the tubes and the sterile filters than the flow channel configuration shown in Fig. 9. In addition, compared to the flow channel configuration shown in Fig. 9, the step shown in Fig. 8C is added.

The flow channel configuration shown in Fig. 9 requires a smaller number of the tubes and the sterile filters than the flow channel configuration shown in Fig. 6, and the liquid feeding procedure can be simplified. On the other hand, after the tube assembly 14 is attached, both of the valves 31B and 31C provided in the middle of the flow channel from the holding part 11 to the connector 90 may be in an open state until the transfer of the suspension is completed, so that the holding part 11 may communicate with the connector 90. Therefore, the connector 90 and the cartridge 80 in a non-sterilized state cannot be used, and it is not possible to perform the liquid feeding in the event of an emergency stop without contaminating the suspension held in the holding part 11.

As described above, the particle detection device 10 according to the embodiment of the disclosed technology includes a holding part 11 that holds a suspension containing particles, a first flow channel (flow channel 20) that is connected to the holding part 11, an observation window 13 that is connected to the first flow channel, a liquid feeding part that transfers the suspension held in the holding part 11 to the observation window 13, and a fluorescence detection part 50 that detects fluorescence emitted from the particles contained in the suspension through the observation window 13.

With the particle detection device 10 according to the embodiment of the disclosed technology, the flow channel from the holding part 11 to the observation window 13 is a closed system, so that the inside of the flow channel can be maintained in a sterile state. In addition, the transfer of the suspension from the holding part 11 to the observation window 13 is performed by the liquid feeding part.

The cell suspension may contain oil droplets derived from a biological tissue in addition to cells. In a case in which the cell suspension is observed using a bright-field microscope that illuminates an object with white light, it is difficult to distinguish between cells and oil droplets contained in the cell suspension. Therefore, it is difficult to count the number of cells contained in the cell suspension using the bright-field microscope. With the particle detection device according to the present embodiment, the fluorescence detection part 50 detects fluorescence emitted from the particles contained in the suspension, making it possible to detect the cells without being affected by the oil droplets contained in the cell suspension. In addition, the particle detection using fluorescence is extremely effective in a case of distinguishing between live cells and dead cells, between cells and other particles, and between cells having a specific protein and other particles. As described above, with the particle detection device 10 according to the present embodiment, it is possible to ensure sterility and to have automation suitability and high particle identification capability.

The particle detection device 10 may include a mixing part 12 in which the suspension flowing through the first flow channel and a fluorescent reagent are mixed. The particle detection device 10 includes the mixing part 12, so that it is possible to perform fluorescence staining of particles contained in the suspension in a closed system.

The mixing part 12 may include a second flow channel (flow channel 21) that is provided between the first flow channel and the observation window 13 and that has a solid-phase fluorescent reagent attached to its inner wall. With the mixing part 12 having the above-described configuration, as the suspension passes through the flow channel 21, the solid-phase fluorescent reagent is dissolved, and the fluorescent reagent and the suspension are mixed. By using the solid-phase fluorescent reagent, it is possible to simplify the device configuration as compared with a case in which a liquid fluorescent reagent is used.

The mixing part 12 may have a form of a cartridge 80 including the observation window 13 and the second flow channel. Since the mixing part 12 has the form of the cartridge 80 that is attachable to and detachable from the flow channel 20, the mixing part 12 can be handled as a single-use member. That is, a new fluorescent reagent can be introduced into the device only by replacing the cartridge 80. As a result, it is not necessary to manage the storage of the fluorescent reagent.

The particle detection device 10 may include a connector 90. The connector 90 has a first port 91 to which the first flow channel is connected and a second port 92 to which the second flow channel is connected, and the cartridge 80 of the mixing part 12 is attachably and detachably attached to the connector 90. The particle detection device 10 includes the connector 90, so that it is possible to easily connect the first flow channel and the second flow channel in the cartridge 80.

The particle detection device 10 may include a valve that opens and closes the first flow channel. Even in a case in which a sterile state cannot be maintained on the downstream side of the valve, the suspension held in the holding part 11 can be maintained in a sterile state by closing the valve.

The particle detection device 10 may include a counting part 60 that counts the number of particles detected by the fluorescence detection part 50. The particle detection device 10 includes the counting part 60, so that it is possible to automate all steps from the extraction of the suspension to the counting of the number of particles.

The particle detection device 10 may include a pump connected to the first flow channel on the upstream side of the mixing part 12, a first valve that opens and closes the first flow channel on the upstream side with respect to a connection point between the first flow channel and the pump, and a second valve that opens and closes the first flow channel on the downstream side with respect to the connection point and on the upstream side with respect to the mixing part 12. With this configuration, it is possible to transfer the suspension to the observation window 13 while maintaining a state where at least one of the first valve or the second valve is closed. That is, liquid can be fed to the observation window without communication between the holding part 11 and the mixing part 12. As a result, even in a case in which the section from the mixing part 12 to the observation window 13 is in a non-sterilized state, a sterile state can be maintained in the suspension held in the holding part 11.

In the above description, a case in which the particle detection device 10 includes the mixing part 12 in which the suspension and the fluorescent reagent are mixed has been exemplified, but, in a case in which particles exhibiting auto-fluorescence are a detection target, the mixing part 12 can be omitted. In addition, in the above description, cells have been exemplified as particles to be detected, but the disclosed technology can be applied to the detection of any particle that can be subjected to fluorescence staining.

The following appendices are further disclosed with respect to the above embodiment.

Appendix 1

A particle detection device comprising:

a holding part that holds a suspension containing particles;
a first flow channel that is connected to the holding part;
an observation window that is connected to the first flow channel;
a liquid feeding part that transfers the suspension held in the holding part to the observation window; and
a fluorescence detection part that detects fluorescence emitted from the particles contained in the suspension through the observation window.

Appendix 2

The particle detection device according to Appendix 1, further comprising:
a mixing part in which the suspension flowing through the first flow channel and a fluorescent reagent are mixed.

Appendix 3

The particle detection device according to Appendix 2,

in which the mixing part has a second flow channel provided between the first flow channel and the observation window, and
a solid-phase fluorescent reagent is attached to an inner wall of the second flow channel.

Appendix 4

The particle detection device according to Appendix 3,
in which the mixing part has a form of a cartridge including the observation window and the second flow channel.

Appendix 5

The particle detection device according to Appendix 4, further comprising:
a connector that has a first port to which the first flow channel is connected and a second port to which the second flow channel is connected, and to which the cartridge of the mixing part is attachably and detachably attached.

Appendix 6

The particle detection device according to any one of Appendices 1 to 5, further comprising:
a valve that opens and closes the first flow channel.

Appendix 7

The particle detection device according to any one of Appendices 2 to 5,

in which the liquid feeding part includes a pump connected to the first flow channel on an upstream side of the mixing part, and
the particle detection device further comprises

a first valve that opens and closes the first flow channel on an upstream side with respect to a connection point between the first flow channel and the pump, and
a second valve that opens and closes the first flow channel on a downstream side with respect to the connection point and on an upstream side with respect to the mixing part.

Appendix 8

The particle detection device according to any one of Appendices 1 to 7, further comprising:
a counting part that counts the number of particles detected by the fluorescence detection part.

Appendix 9

The particle detection device according to any one of Appendices 1 to 8,
in which the particles are cells.

Appendix 10

A particle detection method comprising:

transferring a suspension containing particles held in a holding part to an observation window via a first flow channel using a liquid feeding part; and
detecting fluorescence emitted from the particles contained in the suspension through the observation window.

Appendix 11

The particle detection method according to Appendix 10,
in which the suspension and a fluorescent reagent are mixed in a mixing part connected to the first flow channel.

Appendix 12

The particle detection method according to Appendix 11,

in which the liquid feeding part includes a pump connected to the first flow channel on an upstream side of the mixing part, and
the suspension is transferred to the observation window while at least one of a first valve that opens and closes the first flow channel on an upstream side with respect to a connection point between the first flow channel and the pump or a second valve that opens and closes the first flow channel on a downstream side with respect to the connection point and on an upstream side with respect to the mixing part is maintained in a closed state.

Appendix 13

The particle detection method according to any one of Appendices 10 to 12,
in which the particles are cells.

Explanation of References

1: suspension
10: particle detection device
11: holding part
12: mixing part
13: observation window
13A: chamber

14: tube assembly
20, 21: flow channel
22A, 22B, 22C, 22D, 22E, 22F: tube
23A, 23B: sterile filter
30, 31A, 31B, 31C, 31D: valve
40: liquid feeding part41A, 41B, 41C: pump
50: fluorescence detection part
60: counting part
70: fluorescent reagent
80: cartridge
81: syringe pump
82: protrusion
83: hole portion
90: connector
91: first port
92: second port
93: third port

## Claims

1. A particle detection device comprising:

   a holding part that holds a suspension containing particles;
   a first flow channel that is connected to the holding part;
   an observation window that is connected to the first flow channel;
   a liquid feeding part that transfers the suspension held in the holding part to the observation window; and
   a fluorescence detection part that detects fluorescence emitted from the particles contained in the suspension through the observation window.

2. The particle detection device according to claim 1, further comprising:
   a mixing part in which the suspension flowing through the first flow channel and a fluorescent reagent are mixed.

3. The particle detection device according to claim 2,

   wherein the mixing part has a second flow channel provided between the first flow channel and the observation window, and
   a solid-phase fluorescent reagent is attached to an inner wall of the second flow channel.

4. The particle detection device according to claim 3, wherein the mixing part has a form of a cartridge including the observation window and the second flow channel.

5. The particle detection device according to claim 4, further comprising:

a connector that has a first port to which the first flow channel is connected and a second port to which the second flow channel is connected, and to which the cartridge of the mixing part is attachably and detachably attached.

6. The particle detection device according to any one of claims 1 to 5, further comprising:
   a valve that opens and closes the first flow channel.

7. The particle detection device according to any one of claims 2 to 5,

   wherein the liquid feeding part includes a pump connected to the first flow channel on an upstream side of the mixing part, and
   the particle detection device further comprises

   a first valve that opens and closes the first flow channel on an upstream side with respect to a connection point between the first flow channel and the pump, and
   a second valve that opens and closes the first flow channel on a downstream side with respect to the connection point and on an upstream side with respect to the mixing part.

8. The particle detection device according to any one of claims 1 to 7, further comprising:
   a counting part that counts the number of particles detected by the fluorescence detection part.

9. The particle detection device according to any one of claims 1 to 8,
   wherein the particles are cells.

10. A particle detection method comprising:

    transferring a suspension containing particles held in a holding part to an observation window via a first flow channel using a liquid feeding part; and
    detecting fluorescence emitted from the particles contained in the suspension through the observation window.

11. The particle detection method according to claim 10, wherein the suspension and a fluorescent reagent are mixed in a mixing part connected to the first flow channel.

12. The particle detection method according to claim 11,

    wherein the liquid feeding part includes a pump connected to the first flow channel on an upstream side of the mixing part, and
    the suspension is transferred to the observation window while at least one of a first valve that opens and closes the first flow channel on an upstream side with respect to a connection point between the first flow channel and the pump or a second valve that opens and closes the first flow channel on a downstream side with respect to the connection point and on an upstream side with respect to the mixing part is maintained in a closed state.

13. The particle detection method according to any one of claims 10 to 12, wherein the particles are cells.

# FIG. 1

# FIG. 2

# FIG. 3

## FIG. 4A

## FIG. 4B

## FIG. 4C

## FIG. 5A

## FIG. 5B

## FIG. 5C

# FIG. 5D

# FIG. 5E

# FIG. 6

# FIG. 7

# FIG. 8A

# FIG. 8B

# FIG. 8C

# FIG. 8D

## FIG. 8E

# FIG. 9

# FIG. 10

# FIG. 11A

# FIG. 11B

10

41A
P ON

41B
P OFF

23A

22B
31A
OPEN

22D

22C    31B    31C

31D
CLOSE

CLOSE    OPEN

22A

31E
OPEN

11

21   13   41C
P   80
90    12    OFF

# FIG. 11C

# FIG. 11D

Europäisches
Patentamt

European
Patent Office

Office européen
des brevets

# EUROPEAN SEARCH REPORT

**Application Number**

EP 25 19 0357

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/223161 A1 (CHEN STEVE XIANGLING [US] ET AL) 22 July 2021 (2021-07-22) * paragraphs [0060] - [0363]; figures 29-37A * | 1-13 | INV. G01N1/30 G01N15/10 G01N15/14 |
| X | US 2019/056385 A1 (GERSHTEIN SERGEY [US] ET AL) 21 February 2019 (2019-02-21) * paragraphs [0032] - [0122]; figures 1-4J, 17 * | 1-6, 8-11,13 | |
| X | US 2022/074845 A1 (MUZYKOVSKI VLADIMIR [IL] ET AL) 10 March 2022 (2022-03-10) * paragraphs [0056] - [0160]; figures 1-14 * | 1,8-10, 13 | |
| X | US 2018/095023 A1 (SHI WENDIAN [US] ET AL) 5 April 2018 (2018-04-05) * paragraphs [0032] - [0153]; figures 1A-24A * | 1-6, 8-11,13 | |

**TECHNICAL FIELDS SEARCHED (IPC)**

G01N

The present search report has been drawn up for all claims

| Place of search | Date of completion of the search | Examiner |
|---|---|---|
| The Hague | 7 December 2025 | Ionescu, C |

EPO FORM 1503 03.82 (P04C01)

## ANNEX TO THE EUROPEAN SEARCH REPORT
## ON EUROPEAN PATENT APPLICATION NO.

EP 25 19 0357

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2025

| Patent document cited in search report | Publication date | Patent family member(s) | Publication date |
|---|---|---|---|
| US 2021223161 A1 | 22-07-2021 | AU 2021207987 A1 | 18-11-2021 |
| | | AU 2021254575 A1 | 18-11-2021 |
| | | AU 2022204320 A1 | 07-07-2022 |
| | | AU 2024205076 A1 | 15-08-2024 |
| | | CA 3137050 A1 | 22-07-2021 |
| | | CN 113840924 A | 24-12-2021 |
| | | CN 114410761 A | 29-04-2022 |
| | | CN 115261381 A | 01-11-2022 |
| | | CN 115287341 A | 04-11-2022 |
| | | CN 115369157 A | 22-11-2022 |
| | | DE 112021000050 T5 | 25-05-2022 |
| | | DE 112021000088 B4 | 24-04-2025 |
| | | DE 202021001858 U1 | 05-08-2021 |
| | | EP 3942039 A1 | 26-01-2022 |
| | | EP 3988922 A1 | 27-04-2022 |
| | | EP 4056988 A1 | 14-09-2022 |
| | | EP 4060321 A1 | 21-09-2022 |
| | | EP 4089182 A1 | 16-11-2022 |
| | | GB 2598233 A | 23-02-2022 |
| | | GB 2598498 A | 02-03-2022 |
| | | GB 2604072 A | 24-08-2022 |
| | | GB 2604297 A | 31-08-2022 |
| | | GB 2605310 A | 28-09-2022 |
| | | GB 2615914 A | 23-08-2023 |
| | | GB 2616362 A | 06-09-2023 |
| | | GB 2626884 A | 07-08-2024 |
| | | IL 287375 A | 01-12-2021 |
| | | IL 287430 A | 01-12-2021 |
| | | IL 303164 A | 01-07-2023 |
| | | IL 311540 A | 01-05-2024 |
| | | JP 7223165 B2 | 15-02-2023 |
| | | JP 2022129394 A | 05-09-2022 |
| | | JP 2022540970 A | 21-09-2022 |
| | | KR 20210154868 A | 21-12-2021 |
| | | KR 20220118300 A | 25-08-2022 |
| | | SG 10202112013Q A | 30-12-2021 |
| | | SG 11202111573V A | 29-11-2021 |
| | | US 11053540 B1 | 06-07-2021 |
| | | US 11060138 B1 | 13-07-2021 |
| | | US 2021223161 A1 | 22-07-2021 |
| | | US 2021332416 A1 | 28-10-2021 |
| | | US 2021333210 A1 | 28-10-2021 |
| | | US 2021333211 A1 | 28-10-2021 |
| | | US 2022025457 A1 | 27-01-2022 |
| | | US 2022136047 A1 | 05-05-2022 |
| | | US 2022251643 A1 | 11-08-2022 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 25 19 0357

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2025

| Patent document cited in search report | Publication date | Patent family member(s) | | Publication date |
|---|---|---|---|---|
| | | US 2022251644 A1 | | 11-08-2022 |
| | | US 2022267842 A1 | | 25-08-2022 |
| | | US 2025197925 A1 | | 19-06-2025 |
| | | WO 2021146597 A1 | | 22-07-2021 |
| US 2019056385 A1 | 21-02-2019 | CN 111164411 A | | 15-05-2020 |
| | | EP 3669173 A1 | | 24-06-2020 |
| | | US 2019056385 A1 | | 21-02-2019 |
| | | WO 2019035087 A1 | | 21-02-2019 |
| US 2022074845 A1 | 10-03-2022 | CN 113302492 A | | 24-08-2021 |
| | | EP 3906413 A2 | | 10-11-2021 |
| | | JP 2022516556 A | | 28-02-2022 |
| | | US 2022074845 A1 | | 10-03-2022 |
| | | WO 2020141463 A2 | | 09-07-2020 |
| US 2018095023 A1 | 05-04-2018 | NONE | | |

EPO FORM P0459

For more details about this annex : see Official Journal of the European Patent Office, No. 12/82

page 2 of 2

**EP 4 685 454 A1**

**REFERENCES CITED IN THE DESCRIPTION**

**Patent documents cited in the description**

- JP 7082008 B
- JP H07082008 B
- WO 2016013395 A
- JP 2005241435 A